# EUROPÄISCHE PATENTSCHRIFT

(11) **EP 1 385 449 B1**
(45) Veröffentlichungstag und Bekanntmachung des Hinweises auf die Patenterteilung: **12.07.2006**
(21) Anmeldenummer: 02737972.6
(22) Anmeldetag: 19.04.2002
(51) Int. Cl.: A61F 2/00, A61F 2/28, A61F 2/44, A61L 31/02, A61L 31/04, A61L 31/08, A61L 31/14

(54) **BIOLOGISCH FUNKTIONALISIERTE, METABOLISCH INDUKTIVE IMPLANTATOBERFLÄCHEN**
BIOLOGICALLY-FUNCTIONALISED, METABOLICALLY-INDUCTIVE IMPLANT SURFACES
SURFACES D'IMPLANTS A FONCTIONNALISATION BIOLOGIQUE ET A INDUCTION METABOLIQUE

(30) Priorität: 19.04.2001 DE 10119096
(43) Veröffentlichungstag der Anmeldung: 04.02.2004
(73) Patentinhaber: Mathys AG Bettlach, 2544 Bettlach (CH); INSTITUT FÜR BIOPROZESS- UND ANALYSENMESSTECHNIK e.V., 37308 Heiligenstadt (DE); Surface & Interface Technologies GmbH, 37308 Heilbad Heiligenstadt (DE)
(72) Erfinder: LIEFEITH, Klaus, 37318 Uder (DE); HILDEBRAND, Gerhard, 37318 Lutter (DE); RAHM, Jens, 08412 Werdau (DE); GLIEN, Wilfried, 07639 Bad Klosterlausnitz (DE); KOCH, Manfred, 07318 Saalfeld (DE)
(74) Vertreter: Körfer, Thomas
(86) Internationale Anmeldenummer: PCT/EP2002/004350
(87) Internationale Veröffentlichungsnummer: WO 2002/085250

(56) Entgegenhaltungen:
- EP-A- 0 205 997
- WO-A-02/03894
- WO-A-96/37165
- DE-A- 19 836 985
- US-A- 4 612 160
- US-A- 6 129 928

## Beschreibung

### Hintergrund der Erfindung

Die Möglichkeiten der prothetischen Rehabilitation sowohl in der Oralmedizin als auch in der Orthopädie und Unfallchirurgie sind durch die Einbeziehung von Implantaten wesentlich erweitert worden.

Es lässt sich feststellen, dass die orale Implantologie bereits ein integraler Bestandteil der zahnärztlichen Therapie geworden ist. Hierzu hat auch die Anwendung verbesserter bzw. neuer Operationstechniken beigetragen, die das Indikationsspektrum für implantatgetragenen Zahnersatz deutlich erweitert haben. Nach Angaben des deutschen Zentrums für Orale Implantologie wurden allein in Deutschland im Jahr 1998 bereits 165.000 Implantationen von künstlichen Zähnen auf privater Basis vorgenommen. Derzeit vorliegende Prognosen für das nächste Jahrzehnt sprechen weltweit von einer starken Zunahme der Versorgung mit künstlichen Implantatsystemen in der Dentalmedizin. Die Dentalimplantate bestehen in aller Regel aus Titan, deren Oberflächen punktuell bzw. ganzheitlich mit speziellen Verfahren behandelt werden.

Im Bereich der orthopädischen und unfallchirurgischen Implantologie werden derzeit allein in Deutschland ca. 165.000 Hüftprothesen pro Jahr implantiert, wobei 5-8 % der Patienten bereits nach 10 Jahren im wesentlichen aufgrund aseptischer Prothesenlockerungen wieder reimplantiert werden müssen. D.h., davon sind ca. 30.000 Operationen sogenannte Revisionen, die bei den Krankenkassen mit 11.000 EURO/Operation im Vergleich zu 8.600 EURO/Operation für eine Erstimplantation zu Buche schlagen.

Des weiteren stellen auch implantatinduzierte Infektionen einen nicht unerheblichen Kosten- und Risikofaktor dar. So zeigt die von Malchau und Herberts (18) publizierte "Schweden-Studie" bezüglich des Revisionsrisikos nach 134.056 Primärimplantationen von Hüftgelenkprothesen, dass sich trotz der derzeit häufig angewendeten, prophylaktischen Antibiotika-Therapie (Gentamicingabe an den vorpräparierten lmplantationsort) und anderer Maßnahmen zur Verhinderung tiefer Infektionen, die Zahl der aus diesem Grund notwendigen Revisionen auf ca. 1% beläuft. Europaweit belaufen sich die Revisionsraten von Hüftendoprothesen im Bereich von 2-2.5%, wobei die septischen Lockerungen zur Revision der Implantate nach nur 1-2 Jahren Tragezeit führen. In diesem Zusammenhang beziffern Robertsson et al. (19) die Erstrevisionen im Bereich des totalen Knieersatzes (Schweden, 1988 - 1997) auf ca. 11 %.

Trotz intensivster Anstrengungen im Bereich der Biomaterialforschung bzw. im Bereich der prothetischen Produktentwicklung muss somit festgestellt werden, dass die Implantologie bis heute nicht in der Lage ist, ein Biomaterial dauerhaft und absolut komplikationslos in den menschlichen Organismus zu integrieren. Die Ursache hierfür liegt in dem derzeit noch unzureichendem Verständnis über die ablaufenden, hochkomplexen Wechselwirkungsprozesse zwischen Materialoberflächen und biologischer Umgebung. In diesem Zusammenhang muss davon ausgegangen werden, dass auch für die erfindungsgemäß avisierten Implantatsysteme im Hartgewebekontakt unterschiedliche Kriterien berücksichtigt werden müssen. Zu diesen zählen: Biokompatibilität im Sinne von Struktur- und Oberflächenkompatibilität, biomechanische Stabilität und funktionelle Kraftverteilung im periimplantären Knochen sowie die schnelle Einheilung unter Vermeidung implantatinduzierter Infektionen und die langzeitstabile Gewährleistung eines optimalen Kontaktes der Implantatoberfläche zum umliegenden Knochengewebe.

Da die mechanische Stabilität und Integrität der eingesetzten Werkstoffe inzwischen weitestgehend sichergestellt werden kann, treten die Anforderungen des biologischen Umfeldes mehr und mehr in den Vordergrund des Interesses. Dies bedeutet, dass Implantate in zunehmenden Maße nach dem Verbundwerkstoffkonzept entwickelt werden müssen, wobei die lasttragende Bulk Phase vorwiegend die geforderten biomechanischen Eigenschaften einbringt (Strukturkompatibilität) und die Oberflächenphase die Anforderungen des biologischen Umfeldes (Oberflächenkompatibilität) im Kontakt mit dem Gewebe gewährleistet (33).

### Stand der Technik

Im Bereich des Hartgewebekontaktes (Hüft-, Knie-, Schulter- und Dental- und Wirbelsäulenimplantate sowie Nägel für die Traumatologie) wurde mit unterschiedlichsten Ansätzen (thermische Spritzverfahren einschließlich Plasmaspritzverfahren, elektrochemische Abscheidung, Sol-Gel-Technologien, lonenstrahlsputtern, Laserablation) versucht, das gewünschte Eigenschaftsspektrum durch die Beschichtung eines geeigneten metallischen Grundmaterials mit bioaktiven Komponenten wie Bioglas oder Hydroxylapatit (Hauptbestandteil der anorganischen Knochenphase) zu realisieren. Davon konnten sich kommerziell bisher nur das Plasmaspritzverfahren (1, 2) und die elektrochemische Abscheidung (2, 3, 4) etablieren.

Nach Auswertung der ersten klinischen Langzeituntersuchungen ist jedoch davon auszugehen, dass die bisher als langzeitstabil geltenden plasmagespritzten HAP-Schichten im biologischen Umgebungsmilieu einer partiellen Degradation unterliegen (5, 6). Infolgedessen kommt es einerseits zu unerwünschten Phasenänderungen an der Grenzfläche zum Biosystem; andererseits führt dieser Prozess zum Abkapseln und/oder Abplatzen von vorwiegend kristallinen Schichtbestandteilen (Partikeln). Weiterhin gilt als gesichert, dass die initiale Haftfestigkeit in der Grenzfläche zwischen plasmagespritzter HAP-Schicht und metallischem Grundmaterial mit steigender Liegezeit der Implantate deutlich erniedrigt wird und somit zum vorzeitigen Versagen der Prothese führen kann. Die abgelösten Partikel dieser an sich zwar knochengewebeähnlichen Materialien können zu langanhaltenden Fremdkörperreaktionen führen (7, 8), die die gewünschte Osteointegration des Implantates behindern und eine bereits erfolgte Knochenneubildung osteolytisch beeinflussen (5). Mithin kann von einer langzeitstabilen Verbindung zwischen Grundmaterial und Beschichtung nicht die Rede sein.

Demgegenüber wurde in ersten neueren Untersuchungen von Cooley (9) und Maxian (10) die vergleichsweise hohe Effizienz von vollständig degradierbaren, bioaktiven Schichten, die mittels elektrochemischer Verfahren auf metallischen Grundkörpern aufgebracht wurden, nachgewiesen. Die Auswertung von tierexperimentellen und klinischen Studien führte zu der Schlussfolgerung, dass trotz der schnellen und vollständigen Degradation von hochlöslichen Ca/P-Schichten ein sicheres Knochenanwachsverhalten an die Implantatoberfläche gegeben ist. Nachdem solche elektrochemisch hergestellten, hochlöslichen Beschichtungen ausnahmslos einer chemischen Degradation ohne Partikelfreisetzung unterliegen, kann davon ausgegangen werden, dass der oben beschriebene "long-term risk"-Effekt, der an plasmagespritzten HAP-Schichten auf dentalen Implantaten bereits nach 4-jähriger Implantationsdauer klinisch nachgewiesen wurde, durch die Verwendung schnell degradierender Ca/P-Schichten grundsätzlich vermieden werden kann.

Somit kann zusammenfassend festgestellt werden, dass die sich in der klinischen Anwendung befindlichen, hochlöslichen Ca/P-Beschichtungen langfristig deutlich bessere Ergebnisse als die bisher überwiegend verwendeten plasmagespritzten HAP-Schichten versprechen. Derartige Implantate mit Oberflächenbeschichtungen aus resorbierbaren Ca/P-Phasen sind auch Gegenstand verschiedener Patentveröffentlichungen (20-22, 31, 32).

Trotzdem besteht nach wie vor Bedarf nach Implantaten mit verbesserten Eigenschaften, insbesondere hinsichtlich einer schnelleren und langzeitstabileren Osteointegration. Vor allem bei lasttragenden Implantaten ist hier trotz gewisser Fortschritte noch kein optimales Niveau erreicht worden.

Eingehende Untersuchungen der Erfinder führten sehr eindeutig zu dem Ergebnis, dass die jeweilige Oberflächengestaltung von ansonsten identischen Implantatoberflächen, welche eine Beschichtung mit einer schnell resorbierbaren Ca/P-Phase aufweisen, die Osteointegration weit stärker beeinflussen kann, als bisher angenommen wurde. Insbesondere wurde festgestellt, dass im Vergleich zu herkömmlichen lasttragenden Implantaten, die entweder glatte oder leicht angerauhte bzw. kleinporige Oberflächen aufweisen, Implantatoberflächen mit erfindungsgemäß optimierter Porosität sowie Porengröße und interkonnektierenden Poren einen deutlich weitergehenden Knocheneinbau mit Vaskularisierung und ein besseres mechanisches Makro- und Mikrointerlocking zwischen Implantat und dem neugebildeten Knochengewebe ermöglichen. Die Gewährleistung einer optimalen Offenporigkeit und Porosität der Substratoberfläche ist nach Auffassung der Erfinder mitentscheidend für das Erreichen einer langzeitstabilen Implantatverankerung von lasttragenden Prothesen.

Wie die Erfinder bereits nachweisen konnten, ergibt sich durch die Degradation schnell resorbierbarer Ca/P-Schichten insbesondere auf erfindungsgemäß optimierten offenporigen Implantatstrukturen (Elektrolytbewegung, Diffusionsvorgänge usw.) nach deren Inkorparation in das biologische Milieu u.a. eine Übersättigung mit Calciumionen, Phosphationen und Hydroxylionen im Interface, die bereits nach kurzer Zeit zu Präzipitationseffekten führt, in deren Folge nanokristalliner biogener Knochenapatit direkt auf der Substratoberfläche entsteht. Es ist davon auszugehen, dass insbesondere diese spezifischen Präzipitate als Nukleationskeime für die erforderlichen Mineralisationsprozesse zur Etablierung der ECM wirken (osteokonduktiv) und somit ein verbessertes Knochenanwachsverhalten induzieren. In diesem Zusammenhang wurde nun weiterhin von den Erfindern festgestellt, dass auch die Einbringung von dem biogenen Knochenapatit eigenen Elementen in geeigneter Weise in eine vorzugsweise metallische Substratoberfläche zur Entstehung der bereits obengenannten Nukleationskeime in Form von biogenem Knochenapatit führt und dementsprechend ebenfalls ein verbessertes Zellanwachsverhalten induziert. Somit sind den Erfindern mindestens zwei Konditionierungsmöglichkeiten für metallische Substratoberflächen bekannt, die nach deren Inkorporation ins biologische Milieu zur Simulation zumindest des anorganischen Teils der extrazellulären Matrix (ECM) führen und eine gegenüber einer reinen Metalloberfläche signifikant verbesserte Bioaktivität gewährleisten.

Darüber hinaus wurde von den Erfindern erkannt, daß auf solche offenporige Implantatoberflächen, die vorzugsweise eine kontrolliert resorbierbare Ca/P-Phase aufweisen oder die mit dem biogenen Knochenapatit eigenen Elementen dotiert wurden, besonders effektiv Adhäsions- und oder Signalmoleküle aufgebracht werden können, um den organischen Teil der extrazellulären Matrix biochemisch zu simulieren.

Über die Aktivierung einzelner Zellfunktionen durch oberflächengebundene Signalproteine bzw. -peptide wurde in Experimenten an Modelloberflächen (Glas, Quarz, Polymer, Silizium, Metall, PMMA) berichtet (11-13, 29). Einige Patentveröffentlichungen offenbaren die Einlagerung bzw. Anbindung von wachstumsfördernden Molekülen in bzw. an organischen Polymeren und keramischen Strukturen, z.B. Knochenkeramik, mit relativ großen offenen Poren (23-25) oder in/an eine kleinporige Metall- oder Metalloxidoberfläche (24, 26), erwähnen dagegen in keinem Fall die zusätzliche Induktion von spezifischen Nukleationskeimen durch beispielsweise die Anwendung löslicher Ca/P-Schichten.

DE-A-19 836 985, betrifft ein körperverträgliches Implantet (Brustimplantat) bestehend aus keramischem Material mit einer Porenstruktur, wobei die Porendurchmesser im Bereich von 150 micrometer bis 400 micrometer liegen. Besagtes Implantat wird hierbei mit einer Beschichtung versehen, welche das Einwachsen des Implantats in den Körper und die Ausbildung von Gewebe im Implantat fördert.

WO-A-9 637 165 offenbart poriges Material mit einer Oberfläche, deren Beschaffenheit die kapillare Endothelbildung fördert. Auf dieses Material aufgebracht befindet sich ein Adhäsionmolekül, welches das Einwachsen der Endothelzellen in das poröse Material begünstigt. Aus dieser Druckschrift geht das Vorliegen von interkonnektierenden Poren in einem Porenanteil von 15-50% nicht hervor.

EP-A-205 997 offenbart ein Implantat mit einem Oberflächenüberzug aus Wachstumsfaktoren, wobei die Wachstumsfaktoren kovalent an bindungsaktive Gruppen eines am Grundkörper des Implantats vorliegenden Polymers gebunden sind. Aus der Lehre dieser Druckschrift lassen sich offenbar keinerlei porenartige Strukturen entnehmen.

US-A-6 129 928 offenbart Calciumphosphatbeschichtungen in der Größe von 2-30 micrometer, welche auf metallische Implantate aufgebracht werden. Auf diesen Beschichtungen können Wachstumsfaktoren zur Verbesserung der Integrierfähigkeit innerhalb der Knochenstrukturen absorbiert werden. Obwohl in dieser Druckschrift porenhaltige Materialien mit einer Porengröße von mehr als 100 micrometer genannt werden, gibt es keinerlei Hinweis auf das Vorliegen von interkonnektierenden Poren mit einem Porenanteil von 15-50%.

WO-A-0 203 894 offenbart Prothesen, wobei die Oberfläche des zu implantierenden Materials mit einer porenhaltigen Keramikschicht belegt wird, um eine verbesserte Unterlage für die Knochenanlagerung und das Knochenwachstum zu bieten. Auch wenn es sich hier durchaus um eine biologisch funktionalisierte Beschichtung handelt, gibt es keinen Hinweis auf die konkrete Charakterisierung der Poren.

US-A-4 612 160 offenbart ein Verfahren zur Beschichtung eines Metallsubstrates mit einer porösen Metallschicht, welches unter anderem in der Herstellung von medizinischen Prothesen von Bedeutung ist. Diese Druckschrift enthält jedoch keinerlei Hinweis weder auf das Vorliegen von Adhäsions- oder Signalmolekülen noch auf die Konstellation der Porenstruktur.

Alle diese im Stand der Technik beschriebenen biologisch funktionalisierten Materialien bzw. Vorrichtungen unterscheiden sich in ihren mechanischen Eigenschaften und/oder Oberflächeneigenschaften jedoch deutlich von den erfindungsgemäßen Materialien. Die im Stand der Technik offenbarten metallischen Substrate weisen glatte oder kleinporige Oberflächen auf, die im Vergleich zu den erfindungsgemäßen Oberflächenstrukturen die Osteointegration wenig fördern. Knochenkeramiken und andere hochporöse mineralische Werkstoffe oder organische Polymere allein können keinen hohen mechanischen Belastungen ausgesetzt werden und sind daher für viele Anwendungen und insbesondere für lasttragende Implantate im Hartgewebekontakt nicht geeignet.

Erfindungsgemäß soll dementsprechend der Integrationsprozess eines Hartgewebeimplantates durch die Immobilisierung metabolisch induktiver Peptide auf einer knochenfreundlich strukturierten, offenporigen Implantatoberfläche, vorzugsweise unter Ausnutzung der Leitwirkung von verschiedenen Elementdotierungen und/oder resorbierbaren CaP-Schichtsystemen, die insbesondere zur oberflächennahen Ausbildung von anorganischen Nukleationskeimen führen, beschleunigt und im folgenden eine verbesserte Langzeitstabilität des Implantates garantiert werden.

Dieses Ziel einer schnelleren und langzeitstabileren Osteointegration auch und vorzugsweise bei mechanisch stark belasteten Implantaten wird erfindungsgemäß durch biologisch funktionalisierte Beschichtungen mit den kennzeichnenden Merkmalen des vorliegenden Anspruchs 1 gelöst.

Darüber hinaus kann dem eingangs erwähnten Gesichtspunkt der Vermeidung Implantat-induzierter Infektionen erfindungsgemäß dadurch Rechnung getragen werden, dass geeignete Arzneimittelwirkstoffe, z.B. Antibiotika, zusätzlich an die Implantatoberfläche gebunden und kontrolliert davon freigesetzt werden.

### Beschreibung der Erfindung

Die vorliegende Erfindung stellt eine biologisch funktionalisierte Beschichtung bereit, welche dadurch gekennzeichnet ist, dass auf einer offenporigen Substratoberfläche, die im wesentlichen aus einem anorganischen nicht-mineralischen Material mit offenen interkonnektierenden Poren mit einem Porenanteil von 15-50% besteht, Adhäsions- und/oder Signalmoleküle und/oder funktionelle Derivate oder Fragmente davon in einer Weise, welche die Offenporigkeit der Substratoberfläche im wesentlichen nicht beeinträchtigt, immobilisiert sind.

In einem bevorzugten Aspekt der vorliegenden Erfindung sind neben den Adhäsions- und/oder Signalmolekülen bzw. deren funktionellen Derivaten oder Fragmenten eine oder mehrere kontrolliert resorbierbare Ca/P-Phase(n) auf der offenporigen Substratoberfläche aufgebracht.

In einem zweiten bevorzugten Aspekt stellt die vorliegende Erfindung eine wie oben definierte biologisch funktionalisierte Beschichtung bereit, welche dadurch gekennzeichnet ist, dass dem biogenen Knochenapatit eigene Elemente, wie z.B. Ca, Na, Mg, Mn, P, Si, F etc., direkt in eine vorzugsweise metallische Implantatoberfläche eingebracht sind.

In einem weiteren Aspekt umfassen die biologisch funktionalisierten Beschichtungen der Erfindung ferner an die Implantatoberfläche gebundenene Antibiotika und/oder andere Arzneimittelwirkstoffe, die kontrolliert davon freigesetzt werden können.

Ferner stellt die Erfindung Verfahren zur Herstellung dieser Beschichtungen sowie deren Verwendung als Oberflächenbeschichtung von Implantaten, insbesondere im Hartgewebekontakt, bereit.

Die erfindungsgemäßen Beschichtungen sind "metabolisch induktiv", dies bedeutet im Kontext der vorliegenden Erfindung, dass sie die knochengewebstypische Stoffwechselleistung, wie z.B. die Matrixproduktion von Kollagen Typ I und Osteocalcin sowie die aktive Mineralisierung, stimulieren und/oder den primären Metabolismus (z.B. Atmungsaktivität und Energiegewinnung) relevanter Zellen erhöhen.

Für die vorliegende Erfindung geeignete offenporige Substratoberflächen können in Abhängigkeit vom verwendeten Substratmaterial mit verschiedenen bekannten Methoden des Standes der Technik, einschließlich Sinter- Gieß- Spritz- und Lasertechniken, hergestellt werden. Für metallische Substrate wie Titan, Titanlegierungen, z.B. TiAl6V4, andere Metalle und Metallegierungen, z.B. Stahl, Co, Cr, CoCrMo-Legierungen, eignet sich besonders ein Verfahren, bei dem eine oder mehrere plasmagespritzte, korrosionschemisch optimierte poröse Metallschicht(en), vorzugsweise Titanschicht(en) (TPS), auf dem Grundkörper (Metall bzw. Metallegierung) aufgebracht werden. Porengröße und Porenanteil der Schicht(en) lassen sich nach Wunsch einstellen. Weitere Beispiele für geeignete Grundkörper zur Beschichtung mit erfindungsgemäßen porösen Metallschichten, vorzugsweise TPS-Schichten, sind z.B. belastungsoptimierte Verbundwerkstoffe wie kohlefaserverstärkte Kunststoffe oder kohlefaserverstärktes Titan. Untersuchungen zum Einfluss der Porengröße auf das Einwachsverhalten haben ergeben, dass die minimale Porengröße für ein signifikantes Einwachsen von natürlichem Knochen in der Regel bei 75 - 100 µm liegen wird. Poren einer Größe von ca. 200 µm ermöglichen ein signifikantes Wachstum von Knochengewebe, das auch nach längerer Zeit noch anhält. Poren mit größeren Durchmessern als 500 µm führen nur zu einem unzureichenden Einwachsen von natürlichem Knochen. Untersuchungen zum Einfluss der Porosität auf das knöcherne Einwachsverhalten zeigten darüber hinaus, dass Porositäten unter 15-20% zu keinem nennenswerten Einwachsverhalten führen. Porositäten über 50% ergeben wiederum nur ein unvollständiges Einwachsen von natürlichem Knochen.

Vorzugsweise hat deshalb mindestens die oberste Schicht des Implantatsystems einen Porenanteil von 15-50%, bevorzugter 20-35%, und weist offene, interkonnektierende Poren mit einem Durchmesser von ca. 75 - 300 µm, vorzugsweise 100 - 250 µm, insbesondere 150 - 200 µm, auf. Die Schichtdicke dieser offenporigen Schicht sollte dabei im Bereich zwischen 75-500 µm liegen.

Kontrolliert resorbierbare Ca/P-Phasen, die für die biologisch funktionalisierte Beschichtung der vorliegenden Erfindung einsetzbar sind und die Grundlage für die Ausbildung von Nukleationskeimen nach Implantation der Prothese darstellen, lassen sich prinzipiell nach einem geeigneten Verfahren des Standes der Technik (4, 20-22, 31, 32) herstellen. Bevorzugt sind hierbei elektrochemisch abgeschiedene Ca/P-Phasen, wie z.B. in DE 44 31 862 (4) beschrieben, da sich hier über eine breite Variation der Prozessparameter, z.B. Elektrolytzusammensetzung und -konzentration, Temperatur, Stromstärke etc., die Schichtdicke, der Schichtaufbau, die Schichtzusammensetzung und andere Eigenschaften der Ca/P-Phasen reproduzierbar einstellen lassen. Insbesondere muss jedoch gewährleistet sein, dass die offene Porosität und Porigkeit der zu beschichtenden Materialoberfläche nicht beeinträchtigt wird. Im allgemeinen sind amorphe Schichten schneller resorbierbar als kristalline Schichten, so dass je nach der gewünschten Resorptionszeit gezielt eine Mischung von amorphen oder kristallinen Phasen hergestellt werden kann. Eine "amorphe Phase" im Kontext dieser Anmeldung kann auch einzelne nano- oder mikrokristalline Bereiche einschließen. Unterschiedliche Phasen können sowohl nebeneinander in einer Schicht vorliegen als auch in Schichten übereinander angeordnet sein. Die Gesamtschichtdicke der Ca/P-Phase(n) beträgt vorzugsweise 1-50 µm, besonders bevorzugt 10-20 µm. Während das Ca/P-Verhältnis bei stöchiometrisch reinem Hydroxylapatit 1,67 beträgt und bei natürlichen Knochenmaterialien im Bereich von etwa 1,55 bis 1,63 liegt, eignen sich überraschenderweise Ca/P-Phasen mit deutlich niedrigeren Ca/P-Verhältnissen von etwa 1,0 bis etwa 1,5, vorzugsweise etwa 1,0, bis etwa 1,3, besonders gut zur Verwendung in den Beschichtungen der vorliegenden Erfindung. Allerdings schließt dies nicht aus, dass unter Umständen auch Ca/P-Phasen mit höheren Ca/P-Verhältnissen geeignet sein können. Vorzugsweise wird die Degradationskinetik der Ca/P-Beschichtung für unterschiedliche Knochenqualitäten (jüngere und ältere Patienten, verschiedene Implantationsorte) maßgeschneidert werden. Gewünschtenfalls kann die Ca/P-Phase auch mit Elementen des natürlichen Knochens, z.B. Na, Mg, Mn, Si, F, dotiert werden. Zelltoxisch wirkende Schicht- bzw. Phasenbestandteile, wie beispielsweise CaO (17) sind jedoch zu vermeiden.

Bei einer alternativen Ausführungsform der vorliegenden Erfindung wird die gewünschte Bildung von Nukleationskeimen nach der Implantation des Implantates durch eine Vorkonditionierung der offenporigen, vorzugsweise metallischen Substratoberfläche mit Fremdionen, z.B. Natriumionen, und anschließende Temperung (oxidierender Wärmbehandlungsschritt) erreicht. Diese Oberflächenmodifizierung führt unter physiologischen Bedingungen zur Ausbildung einer nukleationsfördernden Oberflächenschicht, welche beispielsweise im Falle der Behandlung einer Titanoberfläche mit Natriumionen und anschließender Temperung gelartige Natriumtitanat-Strukturen umfaßt, und diese Schicht gewährleistet kurzzeitig nach der Implantation die Ausbildung der bereits obengenannten Nukleationskeime direkt auf der offenporigen Substratoberfläche in Form von biogenem Knochenapatit. Geeignete Techniken zur Realisierung der obengenannten Vorkonditionierung der offenporigen Substratoberfläche sind beispielsweise die Einbringung von Ionen verschiedener Elemente, z.B. Ca, Na, Mg, Mn, P, Si, F, vorzugsweise von Natriumionen, durch Ionenimplantation bzw. durch die Einlagerung der offenporigen Substratoberfläche in eine Lösung dieser Ionen, z.B. in eine NaOH-Lösung, mit anschließender Temperung (oxidierender Wärmebehandlung) in diese Substratoberfläche. Hierbei können die gewünschten Elemente einzeln oder koimplantiert werden und sowohl die Elementdichte und laterale Verteilung als auch die Eindringtiefe der Fremdelemente können mittels lonenimplantation/Plasmaimmersionsionenimplantation variiert werden.

Die erfindungsgemäße offenporige Substratoberfläche, die vorzugsweise eine Beschichtung mit einer oder mehreren resorbierbaren Ca/Phase(n) aufweist und/oder mit Ionen, vorzugsweise Metallionen, dotiert wurde und demgemäß das Potential zur Induzierung von anorganischen Nukleationskeimen besitzt, kann mit adhäsionsstimulierenden Signalmolekülen, z.B. spezifischen Peptidsequenzen, beladen werden.

Für die Auswahl von geeigneten Adhäsions- und/oder Signalpeptiden kann das bereits vorhandene umfangreiche Fachwissen bezüglich der zellulären/molekularen Bausteine und Mechanismen der Zell-Signalprotein-Interaktionen genutzt werden. Die Signalproteine bzw. -peptide (als systemisch zirkulierende Bestandteile, von Zellen lokal produziert oder in der extrazellulären Matrix gespeichert) treten durch die Molekularerkennung über zellmembranständige Rezeptoren in Wechselwirkung mit der Zelle. Für die Bindungsspezifität zwischen Rezeptor und Signalprotein sind die Aminosäuresequenzmotive in den Molekülen der Rezeptoren und Signalproteine verantwortlich. Wichtige Zellfunktionen (z.B. Zell-Zell-Adhäsion, Zell-Substrat-Adhäsion, Proliferation, Differenzierung und Mineralisation) sind durch sequenzmotiv-spezifische Signalproteine selektiv aktivierbar. In diesem Zusammenhang gehen Dee (29) und Puleo (31) davon aus, dass die Osteoblastenadhäsion zu etwa 45% durch integrin-vermittelte Mechanismen vonstatten geht und dass auch Mechanismen, die durch Proteoglycane bzw. Glycosaminoglycane als zelluläre Rezeptoren vermittelt werden, an diesem Adhäsionsprozess beteiligt sind. Beispiele von Sequenzmotiven für Zell-ECM-Adhäsion sind RGD (Arginin-Glycin-Asparaginsäure), RGDf (Arginin-Glycin-Asparaginsäure-D-Phenylalanin), REDRV (Arginin-Glutamat-Aspartat-Arginin-Valin), YIGSR (Tyrosin-Isoleucin-Glycin-Serin-Arginin), VAPG (Valin-Alanin-Prolin-Glycin), VGVAPG (Valin-Glycin-Valin-Alanin-Prolin-Glycin), KQAGDV (Lysin-Glutamin-Alanin-Glycin-Aspartat-Valin) HHLGGAKQAGDV (Histidin-Histidin-Leucin-Glycin-Glycin-Alanin-Lysin-Glutamin-Alanin-Glycin-Aspartat-Valin), KRSR (Lysin-Arginin-Serin-Arginin), FHRRIKA, DGEA, LDV, RKK. An der Kontrolle der Zellfunktionen beteiligte Proteine sind unter anderem Fibrinogen (Fn), Bone Sialoprotein (BSP), Bone Morphogenic Proteins, Osteopontin (OP), Vitronectin, ICAM-1, VCAM, Hormone und Wachstumsfaktoren.

Bei größeren Proteinen besteht die Gefahr, dass durch den Sterilisationsprozess, der vor einer Implantation zwingend erforderlich ist, die funktionell erforderliche native Struktur und dreidimensionale Konfiguration dieser Proteine verloren geht. In neueren Veröffentlichungen (12, 29) wurden dementsprechend die kovalente Anbindung von spezifischen Sequenzmotiven (kurzkettigen Peptiden) an Modelloberflächen untersucht. Es kann nunmehr davon ausgegangen werden, dass wichtige Zellfunktionen wie die Proliferation, Differenzierung und Mineralisation verschiedener Zelltypen durch die Wirkung von kurzkettigen Peptiden wie gewünscht kontrolliert und getriggert werden können. Weiterhin gilt allgemein als nachgewiesen, dass insbesondere die schnelle und stabile Adhäsion vorzugsweise von Osteoblasten an die Implantatoberfläche (Hartgewebeersatz) die eigentliche Voraussetzung für die vorgenannten zellulären Prozesse ist. Aufgrund dessen wurden bisher vorzugsweise integrin-bindende Peptide wie RGD bzw. RGDS an verschiedenen Modelloberflächen immobilisiert. Im allgemeinen konnte eine verbesserte Zelladhäsion (Osteoblasten, Fibroblasten, Chondrozyten) nachgewiesen werden, wobei diese leider nicht nur auf die verbesserte Adhäsion von Osteoblasten beschränkt werden konnte.

Die Erfinder kamen daher zu der Schlußfolgerung, dass eine erfindungsgemäße biologisch induktive Implantatoberfläche vorzugsweise mit einer geeigneten Kombination von Signalmolekülen modifiziert sein sollte, die in vorteilhafter Weise beide vorgenannte Adhäsionsmechanismen zur bevorzugten Adaptierung von Osteoblasten auf künstlichen Implantatoberflächen für den Hartgewebeersatz unterstützt.

Erfindungsgemäß werden somit bevorzugt nicht die natürlichen Proteine der ECM, sondern kurzkettige Peptide sowie deren Derivate und Fragmente davon mit etwa 3-25 Aminosäuren Länge eingesetzt, welche spezifische Sequenzmotive der integrinvermittelten (z.B. RGD oder RGDf) oder der proteoglycan- bzw. glycosaminoglycanvermittelten (z.B. YIGSR, REDRV, RKK, KRSR) Adhäsion und vorzugsweise Motive beider Adhäsionsmechanismen umfassen und im wesentlichen die gleichen oder möglicherweise sogar optimierte funktionelle Eigenschaften und/oder biologische Wirkungen zeigen und darüber hinaus den Vorteil aufweisen, dass diese den erforderlichen Sterilisationsprozess besser als große natürliche Proteine überstehen. Dementsprechend ist davon auszugehen, dass die gewünschte Struktur bzw. Nativität der peptid-funktionalisierten Oberfläche auch nach der Sterilisation erhalten bleibt. Bei der Verwendung von spezifischen Sequenzmotiven kann es von Vorteil sein, die jeweilige spezifische Erkennungssequenz mit weiteren Aminosäuren zu verlängern, um die Erkennungssequenz beispielsweise an Ankergruppen oder andere Moleküle zu koppeln ohne die Sequenzerkennung zu stören. Beispiele für derart modifizierte Derivate von RGD bzw. RGDf sind beispielsweise RGDfK (Arginin-Glycin-Asparaginsäure-D-Phenylalanin-Lysin) und RGDfX (X steht für eine beliebige Aminosäure). RGDfk ist darüber hinaus proteolysestabiler als RGD oder RGDf.

Zur Entwicklung derartiger Peptide und deren Derivate bieten sich beispielsweise die Methoden des Molecular Modelling, d.h., der Entwurf und die Testung von Molekülen durch Computersimulation in einer virtuellen Realität, an. Diese Technologie bietet auch für die Optimierung von Adhäsions- und Signalproteinen zur biologischen Funktionalisierung von Implantatmaterialien eine Reihe von Vorteilen. Erstens sind native Adhäsions- und Signalproteine kostenintensiv und in der Anzahl limitiert. Zweitens sind synthetische Produkte, welche die gleichen funktionellen Sequenzmotive, jedoch eine kürzere Kettenlänge aufweisen, stabiler gegen in vivo Degradation und Denaturierung. Zudem lassen sich die Modifizierung bekannter Molekülstrukturen und das Designen neuer Molekülstrukturen und deren Testung durch Computersimulation viel kostengünstiger und zielsicherer als die normalerweise anfallenden aufwendigen experimentellen Arbeiten durchführen.

Die Immobilisierung der vorgenannten Adhäsions- und/oder Signalmoleküle auf der Ca/P-Phase bzw. direkt auf der offenporigen Substratoberfläche kann mittels van der Waalscher Wechselwirkung, lonenbindung oder kovalenter Bindung erfolgen. Nach bisherigen Erkenntnissen wird eine kovalente Bindung oder lonenbindung voraussichtlich eine bessere biologische Aktivität als die Bindung über van der Waalsche Kräfte ergeben. Besonders vorteilhaft in diesem Zusammenhang erscheint die lokale Aufbringung und kovalente Fixierung der Adhäsions- und/oder Signalmoleküle an der Implantatoberfläche, so dass diese auch nur auf die unmittelbar periimplantäre Zellschicht wirksam werden und unerwünschte Fernwirkungen ausgeschlossen werden.

Die optimale Methode in jedem Einzelfall wird jedoch unter anderem von der Art der beteiligten Bindungspartner, den sterischen Erfordernissen, der gewünschten Bindungsstärke etc. abhängen und kann vom Fachmann unschwer in Routineversuchen bestimmt werden.

Die kovalente Bindung von Proteinen/Peptiden an Trägeroberflächen über sogenannte Ankergruppen ist im Stand der Technik grundsätzlich bekannt, wenn auch meist nicht für die Beschichtung von Implantaten beschrieben. Entsprechende Verfahren können, erforderlichenfalls geeignet modifiziert, in analoger Weise angewandt werden.

Beispielsweise können auf Metalloberflächen durch Silanisierung reaktive Gruppen eingeführt werden, welche dann mit geeigneten Ankergruppen, gegebenenfalls unter Verwendung von silanhaltigen Haftvermittlern, Bindungen eingehen können (vgl. z.B. 14 und 26). Die Peptide werden dabei in der Regel über eine Amidbindung an eine zweite funktionelle Gruppe dieser Ankergruppen gebunden. Die Proteine/Peptide können auch durch Einführung einer zusätzlichen reaktiven Gruppe (z.B. Acrylat) funktionalisiert und durch Reaktion mit einer entsprechenden funktionellen Gruppe der Ankergruppen oder auf der gegebenenfalls funktionalisierten, Substratoberfläche gebunden werden.

Im Rahmen der vorliegenden Erfindung ist die Bindung von Peptiden an Ca/P-Schichten bzw. -Phasen von besonderer Bedeutung. Es ist bekannt, dass Polyanionen, z.B. Peptide, die mehrere Aspartat- und Glutaminsäureste enthalten, stark, jedoch reversibel, an Hydroxylapatit binden; dies ist die Grundlage der Hydroxylapatitchromatographie. Chu und Orgel (15) beschreiben die Verwendung von Oligomeren von Glutaminsäure und D,L-2-Amino-5-phosphonopentansäure als Ankergruppen zur Bindung von Biotin an Hydroxylapatit. Analog dazu lassen sich die gewünschten Adhäsions- und Signalproteine bzw. -peptide unschwer mit diesen oder ähnlichen Oligomeren oder Kombinationen davon über Amidbindungen verknüpfen und an die jeweils vorliegende Ca/P-Phase immobilisieren.

Die Ermittlung weiterer geeigneter Ankergruppen für die Bindung an Ca/P-Phasen oder Metalloberflächen liegt ohne weiteres im Rahmen der Fähigkeiten eines Durchschnittsfachmanns auf diesem Gebiet. Beispielsweise kann es für bestimmte Anwendungen von Vorteil sein, ein- oder mehrlagige Polyelektrolyte, selbstorganisierende Monoschichten, Tetraetherlipide, (Poly)ethylenglcol oder resorbierbare Biopolymere, wie z.B. von Milchsäure, Hyaluronsäure oder Glycolsäure abgeleitete Polymere, als Ankergruppen einzusetzen. Ein Beispiel hierfür wäre die Aufbringung von Calciumphosphat-Dünnschichtfilmen mit darin inkorporierten Peptiden unter Einsatz von sich selbst organisierenden Monoschichten auf die Substratoberfläche. In diesem Zusammenhang sollte auch erwähnt werden, daß die oben beschriebene Dotierung der Substratoberfläche, insbesondere einer metallischen Substratoberfläche, oder der Ca/P-Phase mit Fremdelementen auch der Einführung von reaktionsfähigen Si-Atomen bzw. Si-O-Gruppierungen dienen kann, die dann mit weiteren funktionellen Gruppen und/oder Ankergruppen umgesetzt werden können, um die Signalmoleküle auf der Implantatoberfläche zu immobilisieren.

Ankergruppen können auch dazu verwendet werden, um den Abstand der Adhäsions- und/oder Signalmoleküle von der Substratoberfläche zu optimieren. Untersuchungen zur Bindung der RGD-Zellerkennungssequenz an PMMA (Polymethylmethacrylat)-Oberflächen haben ergeben, dass in diesem Fall ein kritischer Mindestabstand von etwa 3,5 nm erforderlich ist, um eine sterisch erfolgreiche Zelladhäsion vollziehen zu können (12). Die optimalen Abstände werden in Abhängigkeit von der Struktur der Substratoberfläche und den eingesetzten Adhäsions- bzw. Signalpeptiden variieren, können jedoch vom Fachmann unschwer in Routineversuchen bestimmt werden. Die Herstellung von maßgeschneiderten Ankergruppen bestimmter Länge ist für den Fachmann ebenfalls eine Routineangelegenheit. Gerade die oben genannten Peptid-Oligomere lassen sich durch geeignete Wahl der Aminosäure-Monomere und der Anzahl der Monomere und gegebenenfalls durch Kombination mit weiteren Aminosäuren oder Peptiden bestimmter Länge leicht in jeder gewünschten Weise modifizieren.

Ankergruppen lassen sich auch vorteilhaft zur Anbindung und kontrollierten Freisetzung von Antibiotika und/oder anderen Arzneimittelwirkstoffen einsetzen. Erfindungsgemäß verwendbare Antibiotika sind beispielsweise Gentamicin, Rifampin, Mupirocin sowie andere dem Fachmann bekannte, übliche und geeignete Antibiotika.

Ein weiterer relevanter Gesichtspunkt für die optimale Gestaltung und damit Effizienz der erfindungsgemäßen Beschichtung ist auch die geometrisch/räumlich strukturierte Anordung der Adhäsions- und/oder Signalmoleküle auf der Substratoberfläche. Sowohl die Orientierung der Moleküle als auch deren Konformation sowie die Oberflächendichte und Stabilität zum Substratmaterial beeinflussen die zu erwartende positive Aktivierung von Zellfunktionen maßgeblich. Die Optimierung dieser Parameter wird im Idealfall eine Simulation der natürlichen extrazellulären Matrix direkt auf der Implantatoberfläche ergeben. Eine gezielte Vorstrukturierung der Substratoberfläche kann die gewünschte Orientierung oder Anordnung der Adhäsions- und/oder Signalmoleküle unterstützen.

Die folgenden Beispiele sollen die Erfindung näher erläutern, ohne diese jedoch darauf zu beschränken.

### BEISPIEL I

Ein Grundkörper aus einer Titanlegierung, TiAl6V4, wird mit einem plasmagespritzten Titan (TPS)-Zweischichtsystem in einer Gesamtdicke von 200-400 µm belegt. Die erste Schicht, welche porenfrei und dicht aufgebracht ist, sorgt für gute Haftung auf dem lmplantatgrundkörper und wird dann mit einer zweiten plasmagespritzten Schicht belegt, welche einen Porenanteil von etwa 20-35% besitzt und offene interkonnektierende Poren eines mittleren Durchmessers von etwa 100 -250 µm aufweist. Auf diese TPS-Schicht wird dann eine 10-20 µm dicke Ca/P-Phase elektrochemisch nach dem in der DE 44 31862 (4) beschriebenen Verfahren so abgeschieden, dass die Offenporigkeit des TPS-Zweischichtsystems erhalten bleibt. Die gebildete Ca/P-Schicht haftet gut auf der offenporigen metallischen Substratoberfläche. Das Ca/P-Verhältnis dieser Schicht, die im wesentlichen aus Brushit und Monetit besteht, beträgt etwa 1,0-1,20. *In-vitro-*Versuche in simulierter Knochenflüssigkeit gemäß DIN 10993 zeigen, daß die ursprünglich aufgebrachte Ca/P-Schicht unter diesen Bedingungen bereits nach 7 Tagen fast vollständig degradiert und nach 14 Tagen praktisch nicht mehr nachweisbar ist. Bereits nach 2 Degradationstagen findet eine Phasenumbildung auf der Materialoberfläche statt und es kommt zur Präzipitation einer dünnen, nicht vollständig auskristallisierten Apatitschicht mit einem Ca/P-Verhältnis von etwa 1,40-1,50, in die punktuell Elektrolytbestandteile (Na, Si, und S) eingebaut sind.

Zellproliferations- und Zellvitalitätstests mit knochengewebstypischen Zellen (Osteoblasten) und unspezifischen Zellen (3T3-Fibroblasten) auf diesem Material und einem Referenzmaterial ohne Ca/P-Schicht zeigen, dass die schnelle Degradation der ursprünglichen Ca/P-Phase unter gleichzeitiger Präzipitation von phasenveränderten Ca/P-Strukturen (Nukleationskeimen) zur Stimulierung der Zellproliferation, Zellvitalität und Kollagen Typ-I-Synthese bei Osteoblasten beiträgt, während die unspezifischen 3T3-Fibroblasten nicht stimuliert werden.

### BEISPIEL II

Eine Ca/P-beschichtete Implantatoberfläche wird hergestellt wie in Beispiel I beschrieben. Die Peptide RGD und YIGSR werden mit dem Dekamer von Glutaminsäure in einem herkömmlichen Festphasen-Peptidsyntheseschritt verknüpft und anschließend an die Ca/P-Phase adsorbiert. Dabei werden negativ geladene Polypeptide als Linker zur Anbindung von Liganden an die CaP-Oberflächen verwendet. Die so hergestellten biologisch funktionalisierten Beschichtungen werden dann den gleichen Degradationstests und Zellproliferationstests unterworfen wie in Beispiel I beschrieben.

### BEISPIEL III

Eine Materialoberfläche bestehend aus Reintitan wird zur reproduzierbaren Integration (Oberflächendichte, Tiefenverteilung usw.) von Natriumionen (ca. 10¹⁷ Ionen cm⁻²) ionenstrahltechnisch modifiziert (34). Die mit Natriumionen modifizierte Titanoberfläche wurde unter definierten Bedingungen oxidiert und wärmebehandelt, um so homogen ausgebildete Natriumtitanat-Strukturen zu erzeugen. Nach Auslagerung dieser Oberflächenmodifikation in simulierter Körperflüssigkeit (Simulation der Verhältnisse nach der Implantation), die zur Hydrolyse von Na₂TiO₃ und nachfolgend ablaufenden Ionenaustauschreaktionen führt, bildet sich ein ca. 100 nm dicker, nanometerstrukturierter Titanat-Hydrogelfilm an der Materialoberfläche aus. Es konnte nachgewiesen werden, dass obengenannte Phase zum einen eine erhöhte Anzahl von Ti-OH-Gruppen aufweist und zudem auch eine negativ geladene elektrische Doppelschicht ausbildet. Dies führt zu einer signifikant erhöhten HA-Präzipitation und somit zur gewünschten Ausbildung von Nukleationskeimen direkt auf der Titanoberfläche. In diesem Zusammenhang wird vermutet, dass neben den topographischen und physiko-chemischen Oberflächeneigenschaften auch die Viskoelastizität und Nanostruktur des sich bildenden Titanat-Hydrogelfilms eine Mitursache für die signifikant verbesserte Nukleationskapazität dieser Materialoberfläche sein könnte, die nachgewiesenermaßen ausschlaggebend für ein verbessertes Zellanwachsverhalten ist.

### BEISPIEL IV

Eine dotierte Implantatoberfläche wird hergestellt wie in Beispiel III beschrieben und nach im Stand der Technik bekannten Verfahren (z.B. wie in (26) beschrieben) mit reaktiven Gruppen versehen. Die Peptide RGDfX und YIGSR werden an geeignete Ankergruppen, die mit den reaktiven Gruppen auf der Implantatoberfläche eine stabile Bindung eingehen können, gekoppelt und durch Umsetzung mit den genannten reaktiven Gruppen an die lmplantatoberfläche gebunden. Die so hergestellten biologisch funktionalisierten Beschichtungen werden dann den gleichen Degradationstests und Zellproliferationstests unterworfen wie in Beispiel I beschrieben.

Zusammenfassend lässt sich feststellen, dass die erfindungsgemäße, mit Adhäsions- und/oder Signalmolekülen funktionalisierte Oberfläche die Zellfunktionen molekular-selektiv kontrollieren und dementsprechend phänotypische zelluläre Reaktionen (Adhäsion, Proliferation, Migration, Matrixbildung) triggern bzw. optimieren kann. In einer bevorzugten Ausführungsform führt die Kombination einer kontrolliert resorbierbaren Ca/P-Schicht mit integrierten Adhäsions- bzw. Signalmolekülen zu einer beschleunigten und langzeitstabilen Osteointegration der Implantatoberflächen. Die resorbierbare Ca/P-Phase unterstützt dabei insbesondere den Prozess der Knochenmineralisation durch die Bereitstellung von Nukleationszentren insbesondere für die Neubildung der anorganischen Knochenphase. In einer weiteren bevorzugten Ausführungsform werden Nukleationszentren auch durch Modifizierung von vorzugsweise metallischen Substratoberflächen mittels z.B. Ionenimplantation induziert. Die simultane Wirkung der biologischen Komponente und der anorganischen Komponente ergibt so eine neue Qualität positiv bioaktiver und metabolisch induktiver lmplantatoberflächen bzw. Medical Devices.

Die aus dem Stand der Technik bekannten lmplantat-induzierten, bakteriellen Infektionen (septische Prothesenlockerung) können durch die Anbindung bzw. kontrollierte Freisetzung von geeigneten Antibiotika direkt von der Implantatoberfläche vermieden werden.

### Literaturverzeichnis

(1) DE GROOT, KLEIN, WOLKE
   Plasma-sprayed coatings of calcium phosphate
   CRC Press, Boca Raton, Ann Arbor, Boston, 1990
(2) DE GROOT, KLEIN, WOLKE
   Chemistry of calcium phosphate bioceramics
   CRC Handbook of bioactive ceramics, 2, 1996, S. 3-16
(3) BAN, MARUNO
   Morphology and microstructure of electrochemically deposited calcium phosphates in a modified simulated body fluid
   Biomaterials, 19, 1998, 1245-1253
(4) DE 44 31 862 C2
   Verfahren zur Beschichtung von Metall- und Keramikoberflächen mit Hydroxylapatiten
(5) WHEELER
   Eight-year clinical retrospective study of titanium plasma-sprayed and hydroxyapatite - coated cylinder implants
   International Journal of Oral and Maxillofacial Implants, 11, 3, 1996, 340-350 Biomedizinische Technik, 40, 1, 1995, S. 377-378
(6) OSHBORN
   Die biologische Leistung der Hydroxylapatitkeramik-Beschichtung auf dem Femurschaft einer Titanendoprothese - erste histologische Auswertung eines Humanexplantats
   Biomedizinische Technik, 32, 1987, 177-183
(7) EVANS, CLARKE-SMITH
   Studies on the mechanism of cell damage by finely ground hydroxyapatite particles in-vitro
   Clinical Materials, 7, 1991, 241-245
(8) SHENG SUN, HWEI TSUANG, SHONG CHANG
   Effect of hydroxyapatite particle size on myoblasts and fibroblasts
   Biomaterials, 18, 1997, 683-690
(9) COOLEY, VAN DELLEN, BURGESS, WINDELER
   The advantages of coated titanium implants prepared by radiofrequency sputtering from hydroxyapatite
   J. Prosthet. Dent., 67, 1992, 93-100
(10) MAXIAN, ZAWADSKI, DUNN
   Effect of CaP coating resorption and surgical fit on the bone/implant interface Journal of Biomedical Material Research, 28, 1994, 1311-1319
(11) GRONOWICZ, MCCARTHY,
   J. Orthopaedic Res., 14, 1996, 878
(12) KANTLEHNER, FINSINGER, MEYER, SCHAFFNER, JONCZYK, DIEFENBACH, NIES, KESSLER
   Selective RGD-mediated Adhesion of Osteoblasts at Surfaces of Implants Angew. Chem. Int. Ed., 38, 4, 1999, 560-562
(13) MANN, TSAI, WEST
   Modification of surfaces with cell adhesion peptides alters extracellular matrix Deposition
   Biomaterials, 20, 1999, 2281-2286
(14) DE OS 43 21 005
(15) CHU, ORGEL
   Bioconjugate Chem., 8, 1997,103-105
(16) DE 197 55 801 A1
   Mit die Zelladhäsion vermittelnden Peptiden beschichtete Implantate und Verfahren zur ihrer Herstellung
(17) HING, KA, GIBSON, IR, REVELL, PA, BEST, SM, BONFIELD, W.
   Influence of Phase Purity on the in vivo Response to Hydroxyapatite
   Proceedings of the 13^{th} Symposium on Ceramics in Medicine, Bologna, Italy, 22.11.-26.11.2000, 373-376
(18) MALCHAU, H, HERBERTS, P.
   Prognose der totalen Hüftarthroplastik (THA)
   63. Annual Meeting of the American Academy of Orthopaedic Surgeons, Atlanta, USA, Februar 22-26, 1996
(19) ROBERTSSON, O, KNUTSON, K, LEWOLD, S, LIDGREN, L.
   The Swedisch Knee Arthroplasty Register: Outcome with special emphasis on 1988-1997
   Handout Scientific Exhibition AAOS, San Francisco, 2001
(20) EP 08 06 212
   Device for incorporation and release of biological active agents
(21) US 5,958,430
   Thin film composition with biological substance and method of making
(22) WO 99/11202 Biomimetic calcium phosphate implant coatings and methods for making the same
(23) DE 197 06 667
   Knochenersatzmaterial mit einer Oberflächenbelegung mit Peptiden mit RGD-Aminosäuresequenz
(24) DE 195 08 753
   Implantierbare Vorrichtung für Gelenkersatz-, Osteosynthese- oder Dentalzwecke
(25) DE 42 42 889
   Hohlendoprothesen mit Knochenwachstumsfördernder Füllung
(26) WO 99/26674
   Method for immobilizing mediator molecule on inorganic and metal implant material
(27) ONDRACEK, KRAWCZYNSKI
   Biomaterials - a research concept
   In: 8 th SIMCER International Symposium on Ceramics, Rimini, 1992
   Zur direkten Knochenverankerung von zementfreien Hüftendoprothesenschäften
   Med. Orth. Tech., 111, 1991, 65-70
(28) DE 100 06 992
   Verfahren zur Beschichtung eines Implantates und Implantat mit Beschichtung
(29) DEE, ANDERSEN, BIZIOS
   Design and function of novel osteoblast-adhesive peptides for chemical modification of biomaterials
   Journal of Biomed Mater Res, 40, 371-377, 1998
(30) PULEO, BIZIOS
   Mechanisms of fibronectin-mediated attachment of osteoblasts to substrates in vitro
   Bone and Mineral., 18, 215-226, 1992
(31) DE 195 04 386
   Verfahren zur Herstellung einer gradierten Beschichtung aus Calciumphosphaten und Metalloxidphasen auf metallischen Implantaten
(32) US 5,723,038
   Process for producing a gradient coating made of calcium phosphate phases and metal oxide phase on metallic implants
(33) WINTERMANTEL, HA
   Biokompatible Werkstoffe und Bauweisen: Implantate für die Medizin und Umwelt
   Springer Verlag, Berlin, Heidelberg, New York, 1996
(34) PHAM, MAITZ, MATZ, REUTHER, STEINER
   Promoted hydroxyapatite nucleation on titanium ion-implanted with sodium
   Thin Solid Films, 379, 50-56, 2000

## Patentansprüche

1. Biologisch funktionalisierte und metabolisch induktive Beschichtung, wobei auf einer offenporigen Substratoberfläche, die aus einem anorganischen nicht-mineralischen Material mit offenen interkonnektierenden Poren mit einem Porenanteil von 15 - 50 % besteht, Adhäsions- und/oder Signalmoleküle und/oder funktionelle Derivate oder Fragmente davon in einer Weise, welche die Offenporigkeit und Porosität der Substratoberfläche im wesentlichen nicht beeinträchtigt, immobilisiert sind und neben den Adhäsions- und/oder Signalmolekülen bzw. deren funktionellen Derivaten oder Fragmenten (eine) mineralisationsfördernde Phase(n) bzw. Struktur(en) auf bzw. in der Substratoberfläche vorgesehen ist/sind.

2. Beschichtung nach Anspruch 1,
**dadurch gekennzeichnet,**
**daß** eine oder mehrere kontrolliert resorbierbare Ca/P-Phase(n) auf der offenporigen Substratoberfläche aufgebracht ist/sind.

3. Beschichtung nach Anspruch 1,
**dadurch gekennzeichnet,**
**daß** ein oder mehrere Fremdelement(e), das bzw. die aus der Gruppe Na, Ca, P, Mg, Mn, Si, F ausgewählt ist/sind, in die Substratoberfläche eingebracht ist/sind.

4. Beschichtung nach einem der Ansprüche 1 bis 3,
**dadurch gekennzeichnet,**
**daß** die Poren eine Porengröße von 75 - 300 µm aufweisen.

5. Beschichtung nach Anspruch 4,
**dadurch gekennzeichnet,**
**daß** die offenporige Substratoberfläche durch eine Plasmaspritz-, Sinter-, Laser- oder Gießtechnik hergestellt wurde.

6. Beschichtung nach einem der Ansprüche 1 bis 5,
**dadurch gekennzeichnet,**
**daß** die offenporige Substratoberfläche aus einem Metall oder einer Metallegierung besteht.

7. Beschichtung nach Anspruch 6,
**dadurch gekennzeichnet,**
**daß** die offenporige Substratoberfläche eine plasmagespritzte Titanschicht (TPS) ist, die als Einschicht- bzw. Zweischichtsystem ausgebildet wurde.

8. Beschichtung nach einem der Ansprüche 1 bis 7,
**dadurch gekennzeichnet,**
**daß** die Adhäsions- und/oder Signalmoleküle zelluläre Rezeptoren aus der Gruppe der Integrine und Glycosaminoglycane ansprechen.

9. Beschichtung nach einem der Ansprüche 1 bis 8,
**dadurch gekennzeichnet,**
**daß** die Adhäsions- und/oder Signalmoleküle bzw. deren funktionelle Derivate oder Fragmente Peptide umfassen, die beispielsweise eines oder mehrere der Sequenzmotive RGD, RGDf, RGDfK, RGDfX, REDRV, YIGSR, RKK, LDV, VAPG, VGVAPG, KQAGDV, HLGGAKQAGDV, KRSR, KRSRGGG, FHRRIKA aufweisen.

10. Beschichtung nach Anspruch 8 oder 9,
**dadurch gekennzeichnet,**
**daß** die Peptide eine Länge von 3 bis 25 Aminosäuren aufweisen.

11. Beschichtung nach einem der Ansprüche 1, 2, 4 bis 10,
**dadurch gekennzeichnet,**
**daß** die Adhäsions- und/oder Signalmoleküle bzw. deren funktionelle Derivate oder Fragmente auf der Ca/P-Phase immobilisiert sind.

12. Beschichtung nach Anspruch 11,
**dadurch gekennzeichnet,**
**daß** die Ca/P-Schicht(en) ein Ca/P-Verhältnis von etwa 1,0 bis etwa 1,8 aufweist(en).

13. Beschichtung nach einem der Ansprüche 1, 2, 4 bis 12,
**dadurch gekennzeichnet,**
**daß** die Ca/P-Schicht zusätzlich mit einem oder mehreren Element(en), das bzw. die aus der Gruppe aus Na, Mg, Mn, Si, F ausgewählt ist/sind, dotiert ist.

14. Beschichtung nach einem der Ansprüche 1, 2, 4 bis 13,
**dadurch gekennzeichnet,**
**daß** die Ca/P-Beschichtung eine Schichtdicke von 1 - 50 µm aufweist.

15. Beschichtung nach einem der Ansprüche 1, 2, 4 bis 14,
**dadurch gekennzeichnet,**
**daß** sie als geometrische Struktur, die aus Adhäsions- und/oder Signalmolekülen bzw. deren funktionellen Derivaten oder Fragmenten und Ca/P-Phasen aufgebaut ist, auf der Substratoberfläche vorliegt.

16. Beschichtung nach einem der Ansprüche 1, 3 bis 10,
**dadurch gekennzeichnet,**
**daß** das bzw. die Fremdelement(e) mittels Ionenimplantation /Plasmaimmersionsionenimplantation oder Einlagerung der Substratoberfläche in geeignete Elektrolyte in die Substratoberfläche eingebracht ist/sind.

17. Beschichtung nach Anspruch 16,
**dadurch gekennzeichnet,**
**daß** in eine Titanoberfläche Natriumionen eingebracht sind.

18. Beschichtung nach Anspruch 17,
**dadurch gekennzeichnet,**
**daß** sie Natriumtitanatstrukturen umfaßt.

19. Beschichtung nach einem der Ansprüche 1 bis 18,
**dadurch gekennzeichnet,**
**daß** sie auf einer vorstrukturierten Substratoberfläche aufgebracht ist.

20. Beschichtung nach einem der Ansprüche 1 bis 19,
**dadurch gekennzeichnet,**
**daß** die Adhäsions- und/oder Signalmoleküle bzw. deren funktionellen Derivate oder Fragmente über Ankergruppen mit der Substratoberfläche und/oder der Ca/P-Phase verknüpft sind.

21. Beschichtung nach Anspruch 20,
**dadurch gekennzeichnet,**
**daß** es sich bei den Ankergruppen um ein- oder mehrlagige Polyelektrolyte, Tetraetherlipide, selbst organisierende Monoschichten, resorbierbare Biopolymere oder eine 2-Amino-5-Phosphonoalkansäure oder Derivate davon handelt.

22. Beschichtung nach Anspruch 20 oder 21,
**dadurch gekennzeichnet,**
**daß** es sich bei den Ankergruppen um Oligomere von Glutaminsäure, Asparaginsäure, Milchsäure, Hyaluronsäure, Glycolsäure, Ethylenglycol oder um D,L-2-Aminophosphonopentansäure oder Derivate davon handelt.

23. Beschichtung nach einem der Ansprüche 20 bis 22,
**dadurch gekennzeichnet,**
**daß** die Ankergruppen auch die Funktion von Spacern erfüllen.

24. Beschichtung nach einem der Ansprüche 1 bis 23,
**dadurch gekennzeichnet,**
**daß** in die Beschichtung auch Antibiotika und/oder andere Arzneimittelwirkstoffe inkorporiert sind.

25. Beschichtung nach Anspruch 24,
**dadurch gekennzeichnet,**
**daß** die Antibiotika und/oder anderen Arzneimittelwirkstoffe über Ankergruppen an die Substratoberfläche und/oder die Ca/P-Phase gekoppelt sind und gegebenenfalls kontrolliert davon freisetzbar sind.

26. Verfahren zur Oberflächenmodifizierung einer metallischen Substratoberfläche,
wobei ein oder mehrere Fremdelement(e), das/die aus der Gruppe aus Na, Ca, P, Mg, Mn, Si, F ausgewählt ist/sind, mittels Ionenimplantation/Plasmaimmersionsionenimplantation oder Einlagerung der Substratoberfläche in geeignete Elektrolyte in die metallische Substratoberfläche eingebracht wird/werden und die Substratoberfläche anschließend einer oxidierenden Wärmebehandlung unterworfen wird.

27. Verfahren nach Anspruch 26,
**dadurch gekennzeichnet,**
**daß** die Dichte, laterale Verteilung und Eindringtiefe des Fremdelements bzw. der Fremdelemente mittels Ionenimplantation/Plasmaimmersionsionenimplantation eingestellt werden.

28. Verfahren nach Anspruch 26 oder 27,
**dadurch gekennzeichnet,**
**daß** mehrere Fremdelemente nacheinander oder gleichzeitig eingebracht werden.

29. Verfahren nach einem der Ansprüche 26 bis 28,
**dadurch gekennzeichnet,**
**daß** in eine Titanoberfläche Natriumionen eingebracht werden.

30. Verfahren nach einem der Ansprüche 26 bis 29,
**dadurch gekennzeichnet,**
**daß** es sich um eine Substratoberfläche mit offenen interkonnektierenden Poren in einem Porenanteil von 15 - 50 % handelt.

31. Substratoberfläche, erhältlich nach dem Verfahren nach einem der Ansprüche 26 bis 30.

32. Verwendung der Beschichtung nach einem der Ansprüche 1 bis 25 oder der Substratoberfläche nach Anspruch 31 für Implantatoberflächen, insbesondere von Dental- oder Gelenkimplantaten.

33. Implantat mit einer biologisch funktionalisierten Beschichtung nach einem der Ansprüche 1 bis 25 oder mit einer Substratoberfläche nach Anspruch 31.

34. Implantat nach Anspruch 33,
**dadurch gekennzeichnet,**
**daß** es sich um Implantate im Knochenkontakt, z.B. Dental-, Gelenk- oder Wirbelsäulenimplantate bzw. Nägel für die Traumatologie handelt.

## Claims

1. Biologically functionalized and metabolically inductive coating, wherein adhesion and/or signal molecules and/or functional derivatives or fragments thereof are immobilized on an open-pored substrate surface, which comprises an inorganic non-mineral material having open interconnecting pores with a pore content of 15 - 50 %, in a manner which substantially does not impair the open-pored nature and porosity of the substrate surface, and in addition to the adhesion and/or signal molecules or functional derivatives or fragments thereof, (a) mineralization-promoting phase(s) or structure(s) is/are provided on or in the substrate surface.

2. Coating according to claim 1,
**characterized in that**
one or more Ca/P phase(s) which can be absorbed in a controlled manner is/are applied to the open-pored substrate surface.

3. Coating according to claim 1,
**characterized in that**
one or more foreign element(s) which is/are chosen from the group consisting of Na, Ca, P, Mg, Mn, Si and F is/are incorporated into the substrate surface.

4. Coating according to one of claims 1 to 3,
**characterized in that**
the pores have a pore size of 75-300 µm.

5. Coating according to claim 4,
**characterized in that**
the open-pored substrate surface has been prepared by a plasma spraying, sintering, laser or casting technique.

6. Coating according to one of claims 1 to 5,
**characterized in that**
the open-pored substrate surface is made of a metal or a metal alloy.

7. Coating according to claim 6,
**characterized in that**
the open-pored substrate surface is a plasma-sprayed titanium layer (TPS) which has been formed as a single-layer or two-layer system.

8. Coating according to one of claims 1 to 7,
**characterized in that**
the adhesion and/or signal molecules respond to cell receptors from the group consisting of integrins and glycosaminoglycans.

9. Coating according to one of claims 1 to 8,
**characterized in that**
the adhesion and/or signal molecules or functional derivatives or fragments thereof include peptides which have, for example, one or more of the sequence motifs RGD, RGDf, RGDfK, RGDfX, REDRV, YIGSR, RKK, LDV, VAPG, VGVAPG, KQAGDV, HLGGAKQAGDV, KRSR, KRSRGGG or PHRRIKA.

10. Coating according to claim 8 or 9,
**characterized in that**
the peptides have a length of from 3 to 25 amino acids.

11. Coating according to one of claims 1, 2, 4 to 10,
**characterized in that**
the adhesion and/or signal molecules or functional derivatives or fragments thereof are immobilized on the Ca/P phase.

12. Coating according to claim 11,
**characterized in that**
the Ca/P layer(s) has/have a Ca/P ratio of from about 1.0 to about 1.8.

13. Coating according to one of claims 1, 2, 4 to 12,
**characterized in that**
the Ca/P layer is additionally doped with one or more element(s) which is/are chosen from the group consisting of Na, Mg, Mn, Si and F.

14. Coating according to one of claims 1, 2, 4 to 13,
**characterized in that**
the Ca/P coating has a layer thickness of 1 - 50 µm.

15. Coating according to one of claims 1, 2, 4 to 14,
**characterized in that**
it is in the form of a geometric structure, which is built up from adhesion and/or signal molecules or functional derivatives or fragments thereof and Ca/P phases, on the substrate surface.

16. Coating according to one of claims 1, 3 to 10,
**characterized in that**
the foreign element(s) is/are incorporated into the substrate surface by means of ion implantation/plasma immersion implantation or immersion of the substrate surface in suitable electrolytes.

17. Coating according to claim 16,
**characterized in that**
sodium ions are incorporated into a titanium surface.

18. Coating according to claim 17,
**characterized in that**
it comprises sodium titanate structures.

19. Coating according to one of claims 1 to 18,
**characterized in that**
it is applied to a prestructured substrate surface.

20. Coating according to one of claims 1 to 19,
**characterized in that**
the adhesion and/or signal molecules or functional derivatives or fragments thereof are linked with the substrate surface and/or the Ca/P phase via anchor groups.

21. Coating according to claim 20,
**characterized in that**
the anchor groups are single- or multilayered polyelectrolytes, tetraether lipids, self-organizing monolayers, absorbable biopolymers or a 2-amino-5-phosphonoalkanoic acid or derivatives thereof.

22. Coating according to claim 20 or 21,
**characterized in that**
the anchor groups are oligomers of glutamic acid, aspartic acid, lactic acid, hyaluronic acid, glycolic acid or ethylene glycol, or D,L-2-aminophosphonopentanoic acid or derivatives thereof.

23. Coating according to one of claims 20 to 22,
**characterized in that**
the anchor groups also fulfil the function of spacers.

24. Coating according to one of claims 1 to 23,
**characterized in that**
antibiotics and/or other medicament active compounds are also incorporated into the coating.

25. Coating according to claim 24,
**characterized in that**
the antibiotics and/or other medicament active compounds are coupled to the substrate surface and/or the Ca/P phase via anchor groups and optionally can be released therefrom in a controlled manner.

26. Process for the surface modification of a metallic substrate surface,
wherein one or more foreign element(s), which is/are chosen from the group consisting of Na, Ca, P, Mg, Mn, Si and F, is/are incorporated into the metallic substrate surface by means of ion implantation/plasma immersion implantation or immersion of the substrate surface in suitable electrolytes and the substrate surface is then subjected to an oxidizing heat treatment.

27. Process according to claim 26,
**characterized in that**
the density, lateral distribution and depth of penetration of the foreign element or foreign elements are adjusted by means of ion implantation/plasma immersion implantation.

28. Process according to claim 26 or 27,
**characterized in that**
several foreign elements are incorporated successively or simultaneously.

29. Process according to one of claims 26 to 28,
**characterized in that**
sodium ions are incorporated into a titanium surface.

30. Process according to one of claims 26 to 29,
**characterized in that**
the substrate surface is one which has open interconnecting pores in a pore content of 15 - 50 %.

31. Substrate surface obtainable by the process according to one of claims 26 to 30.

32. Use of the coating according to one of claims 1 to 25 or of the substrate surface according to claim 31 for implant surfaces, in particular of dental or joint implants.

33. Implant having a biologically functionalized coating according to one of claims 1 to 25 or having a substrate surface according to claim 31.

34. Implant according to claim 33,
**characterized in that**
it is an implant in contact with bone, e.g. dental, joint or vertebral column implants or nails for traumatology.

## Revendications

1. Revêtement biologiquement fonctionnalisé et métaboliquement inductif, dans lequel des molécules d'adhésion et/ou des molécules signal et/ou des dérivés ou fragments fonctionnels de celles-ci sont immobilisés sur une surface de substrat à pores ouverts constituée d'un matériau non minéralisé anorganique avec des pores interconnectants ouverts dans une proportion de pores de 15 à 50 %, de telle façon que l'ajouration et la porosité de la surface de substrat ne soit pas altérée de manière importante, et, à côté des molécules d'adhésion et/ou des molécules signal, respectivement de leurs dérivés ou fragments fonctionnels, une (des) phase(s), respectivement une (des) structure(s), favorisant la minéralisation sur, respectivement dans, la surface de substrat est(sont) prévue(s).

2. Revêtement selon la revendication 1,
**caractérisé en ce qu'**
on applique une ou plusieurs phase(s) Ca/P résorbable(s) de manière contrôlée sur la surface de substrat à pores ouverts.

3. Revêtement selon la revendication 1,
**caractérisé en ce qu'**
un ou plusieurs élément(s) étranger(s), qui est (sont) choisi(s) dans le groupe Na, Ca, P, Mg, Mn, Si, F, est (sont) incorporés dans la surface de substrat.

4. Revêtement selon l'une quelconque des revendications 1 à 3,
**caractérisé en que**
les pores présentent une taille de pore de 75 à 300 µm.

5. Revêtement selon la revendication 4,
**caractérisé en ce que**
la surface de substrat à pores ouverts a été fabriquée par une technique de projection au plasma, de frittage, laser ou coulée.

6. Revêtement selon l'une quelconque des revendications 1 à 5,
**caractérisé en ce que**
la surface de substrat à pores ouverts est constituée d'un métal ou d'un alliage de métal.

7. Revêtement selon la revendication 6,
**caractérisé en ce que**
la surface de substrat à pores ouverts est une couche de titane projetée au plasma (TPS), qui a été constituée en tant que système à une seule couche respectivement à deux couches.

8. Revêtement selon l'une quelconque des revendications 1 à 7,
**caractérisé en ce que**
les molécules d'adhésion et/ou les molécules signal s'adressent à des récepteurs cellulaires du groupe des intégrines et glycosaminoglycanes.

9. Revêtement selon l'une quelconque des revendications 1 à 8,
**caractérisé en ce que**
les molécules d'adhésion et/ou le molécules signal, respectivement leurs dérivés ou fragments fonctionnels, renferment des peptides qui présentent, par exemple, un ou plusieurs des motifs de séquence RGD, RGDf, RGDfK, RGDfX, REDRV, YIGSR, RKK, LDV, VAPG, VGVAPG, KQAGDV, HLGGAKQAGDV, KRSR, KRSRGGG, FHRRIKA.

10. Revêtement selon la revendication 8 ou 9,
**caractérisé en ce que**
les peptides ont une longueur de 3 à 25 acides aminés.

11. Revêtement selon l'une quelconque des revendications 1, 2, 4 à 10,
**caractérisé en ce que**
les molécules d'adhésion et/ou les molécules signal, respectivement leurs dérivés ou fragments fonctionnels, sont immobilisés sur la phase Ca/P.

12. Revêtement selon la revendication 11,
**caractérisé en ce que**
la (les) couche(s) Ca/P présente(nt) un rapport CA/P d'environ 1,0 à environ 1,8.

13. Revêtement selon l'une quelconque des revendications 1, 2, 4 à 12,
**caractérisé en ce que**
la couche Ca/P est dotée, en complément, d'un ou plusieurs élément(s) choisi(s) dans le groupe constitué de Na, Mg, Mn, Si, F.

14. Revêtement selon l'une quelconque des revendications 1, 2, 4 à 13,
**caractérisé en ce que**
le revêtement Ca/P a une épaisseur de couche de 1 à 50 µm.

15. Revêtement selon l'une quelconque des revendications 1, 2, 4 à 14,
**caractérisé en ce qu'**
il se présente, sur la surface de substrat, en tant que structure géométrique constituée de molécules d'adhésion et/ou de molécules signal, respectivement de leurs dérivés ou fragments fonctionnels, et de phases Ca/P.

16. Revêtement selon l'une quelconque des revendications 1, 3 à 10,
**caractérisé en ce que**
l'élément, respectivement les éléments étranger(s), est (sont) introduits dans la surface de substrat par implantation ionique/implantation ionique par immersion plasma ou stockage de la surface de substrat dans des électrolytes appropriés.

17. Revêtement selon la revendication 16,
**caractérisé en ce que**
des ions de sodium sont introduits dans une surface de titane.

18. Revêtement selon la revendication 17,
**caractérisé en ce qu'**
il renferme des structures de titanate de sodium.

19. Revêtement selon l'une quelconque des revendications 1 à 18,
**caractérisé en ce qu'**
il est appliqué sur une surface de substrat pré-structurée.

20. Revêtement selon l'une quelconque des revendications 1 à 19,
**caractérisé en ce que**
les molécules d'adhésion et/ou les molécules signal, respectivement leurs dérivés ou fragments fonctionnels, sont reliés à la surface de substrat et/ou à la phase Ca/P par l'intermédiaire de groupes d'ancrage.

21. Revêtement selon la revendication 20,
**caractérisé en ce que**
les groupes d'ancrage sont des polyélectrolytes à une ou plusieurs couches, des tétraétherlipides, des monocouches s'organisant elles-mêmes, des biopolymères résorbables ou un acide de 2-amino-5-phosphonoalcane ou des dérivés de ces substances.

22. Revêtement selon la revendication 20 ou 21,
**caractérisé en ce que**
les groupes d'ancrage sont des oligomères de l'acide glutaminique, de l'acide asparagique, de l'acide lactique, de l'acide hyaluronique, de l'acide glycolique, de l'éthylène glycol ou de l'acide de D,L-2-aminophosphonopentane ou des dérivés de ces substances.

23. Revêtement selon l'une quelconque des revendications 20 à 22,
**caractérisé en ce que**
les groupes d'ancrage assurent également la fonction d'espaceurs.

24. Revêtement selon l'une quelconque des revendications 1 à 23,
**caractérisé en ce que**
des antibiotiques et/ou d'autres agents actifs médicamenteux sont également incorporés au revêtement.

25. Revêtement selon la revendication 24,
**caractérisé en ce que**
les antibiotiques et/ou d'autres agents actifs médicamenteux sont couplés à la surface de substrat et/ou à la phase Ca/P par l'intermédiaire de groupes d'ancrage et, le cas échéant, peuvent en être libérés de manière contrôlée.

26. Procédé de modification superficielle d'une surface de substrat métallique,
dans lequel un ou plusieurs élément(s) étranger(s), choisi(s) dans le groupe constitué de Na, Ca, P, Mg, Mn, Si, F, est (sont) incorporé(s) dans la surface de substrat métallique par implantation ionique/implantation ionique par immersion plasma ou incorporation de la surface de substrat dans des électrolytes appropriés et la surface de substrat est ensuite soumise à un traitement thermique oxydant.

27. Procédé selon la revendication 26,
**caractérisé en ce que**
la densité, la répartition latérale et la profondeur de pénétration de l'élément étranger, respectivement des éléments étrangers, sont ajustées par implantation ionique/implantation ionique par immersion plasma.

28. Procédé selon la revendication 26 ou 27,
**caractérisé en ce que**
plusieurs éléments étrangers sont incorporés l'un après l'autre ou simultanément.

29. Procédé selon l'une quelconque des revendications 26 à 28,
**caractérisé en ce que**
des ions de sodium sont incorporés dans une surface de titane.

30. Procédé selon l'une quelconque des revendications 26 à 29,
**caractérisé en ce qu'**
il s'agit d'une surface de substrat ayant des pores interconnectants ouverts dans une proportion de pores de 15 à 50 %.

31. Surface de substrat, pouvant être obtenue avec le procédé selon l'une quelconque des revendications 26 à 30.

32. Utilisation du revêtement selon l'une quelconque des revendications 1 à 25 ou de la surface de substrat selon la revendication 31 pour des surfaces d'implants, en particulier d'implants dentaires ou articulaires.

33. Implant comportant un revêtement biologiquement fonctionnalisé selon l'une quelconque des revendications 1 à 25 ou ayant une surface de substrat selon la revendication 31.

34. Implant selon la revendication 33,
**caractérisé en ce qu'**
il s'agit d'implants en contact avec les os, par exemple des implants dentaires, articulaires ou dans la colonne vertébrale, respectivement des clous, pour le domaine de la traumatologie.
